Europäisches Patentamt

**European Patent Office**

Office européen des brevets

(19)

(11) Publication number: **0 198 821**
**B1**

(12) **EUROPEAN PATENT SPECIFICATION**

(45) Date of publication of patent specification: **05.10.88**

(51) Int. Cl.⁴: **C 12 M 3/04**

(21) Application number: **84903958.1**

(22) Date of filing: **29.10.84**

(86) International application number:
**PCT/EP84/00340**

(87) International publication number:
**WO 86/02664 09.05.86 Gazette 86/10**

(54) **METHOD FOR THE MASS GROWTH OF CELLS AND APPARATUS FOR THE CARRYING OUT THEREOF.**

(43) Date of publication of application:
**29.10.86 Bulletin 86/44**

(45) Publication of the grant of the patent:
**05.10.88 Bulletin 88/40**

(84) Designated Contracting States:
**AT BE CH DE FR GB LI LU NL SE**

(56) References cited:
**FR-A-1 526 192**
**FR-A-2 311 845**

**Chem. Ing. Techn., vol. 50, no. 6, 1978, (Weinheim, DE), H.W.D. Katinger et al.: "Der Blasensäulenfermenter für die Massensuspensionskultur tierischer Zellen", pp. 472-473**

(73) Proprietor: **LABOR FÜR EXPERIMENTELLE CHIRURGIE, SCHWEIZERISCHES FORSCHUNGSINSTITUT**
**Obere Strasse 22**
**CH-7270 Davos (CH)**

(72) Inventor: **TEPIC, Slobodan**
**Oberstrasse 55**
**CH-7270 Davos Platz (CH)**

(74) Representative: **Lusuardi, Werther G.**
**Dr. Lusuardi AG Stockerstrasse 8**
**CH-8002 Zürich (CH)**

Courier Press, Leamington Spa, England.

## Description

This invention relates to a method for the mass growth of anchorage-dependent cells and an apparatus for the carrying out thereof.

In vitro cell growth is required for many purposes, for instance for the production of vaccines and cell products, for human use and for research purposes.

In in vitro cultures most cells will not divide unless they attach to a solid surface and spread out. This requirement is commonly referred to as "anchorage" requirement. Cells growing on such cell-receptive surfaces adhere firmly to it and form a closed, dense, cell layer (monolayer). The principle of the growth of cells on the surface of a substance therefore establishes a natural limit for production due to the limited size of surface available for culture. For several years it has been attempted to find a practical solution for this limitation by enlarging artificially the surface available for the cell growth, as disclosed for example in the British Patent Specification No. 1,525,022.

It can be stated that none of these known systems for the mass growth of anchorage-dependent cells has reached the stage of routine operational production of anchorage-dependent cells or of cell products. From a practical standpoint, a true mass growth of anchorage-dependent cells has not been obtained with any system, and from a theoretical standpoint all the systems have serious disadvantages. For instance the possibility of microbic and cellular contamination, and the difficulty of rationalization and automatization.

The invention as claimed is intended to provide a remedy. It solves the problem how to produce a true mass growth of cells without the need of a solid surface and more particularly how to design an apparatus for carrying out this process in an industrial manner with the possibility of automatization and rationalization of the process.

It has surprisingly been found that the crucial effect of "anchoring" is the change in shape of the cell, i.e. the spreading out, .rather than the cell's physical contact with a solid surface. Provided that all other conditions for growth (suitable growth medium, temperature, oxygen a.s.o.) are satisfied a flattened-out cell will divide no matter how the flattening was effected.

The method practiced with the present invention allows for spreading out cells within thin films formed by the growth medium itself. In addition to fulfilling the basic requirements of cell flattening, the method offers superior conditions for metabolic exchange with the environment, likely to result in higher growth rates, and hence higher, yields in large scale cell culturing. The method is particularly well suited for continuous growing of cells for the ease of monitoring and control.

The present invention, therefore, has far reaching implications in the field of large scale cell growing.

In the preferred embodiment of the invention cells are grown in the walls of bubbles formed from the growth medium containing cells, much like the familiar soap bubbles, preferably, by a continuous bubble production process. Addition of foetal calf serum, which is still required for most cell lines makes the growth medium suitable for bubble making. The nutrient medium in the bubbles is replenished simply by adding fresh medium in the bubble circulation path. Cell population is kept at optimum by a cell removal rate equal to the growth rate. Life time of the bubble can be much longer than the recirculation time. Recirculation of the bubble should not allow a cell to round up once it was spread out during "bubble time".

The amount of medium at disposal of each cell in the bubble wall is small, but replenishment at ressuspension in the bubble circulation process is sufficient for the next bubble cycle. In contrast to anchored cells, all of the cell's surface is available for metabolic exchanges. Oxygen and carbondioxide exchanges in particular, are far superior to those obtainable in suspension (with microsphere anchorage) cultures.

These and other objects and advantages of the invention will become apparent from the following detailed description taken in conjunction with the accompanying drawings wherein:

Figure 1 (a to e) illustrates the basic principle of the invention by means of sections through cells of different wall shape;

Figure 2 shows a perspective view of a bubble of nutrient medium containing cells;

Figure 3 illustrates a perspective view of a foam of nutrient medium containing cells;

Figure 4 is a perspective view of a solid frame supporting a thin film of nutrient medium;

Figure 5 is a schematic representation of a growth chamber for producing and maintaining of bubble cell colonies;

Figure 6 shows a schematic view of a chamber for cell incubation in foam films;

Figure 7 is a schematic view of a laboratory scale apparatus for cell incubation in foam films.

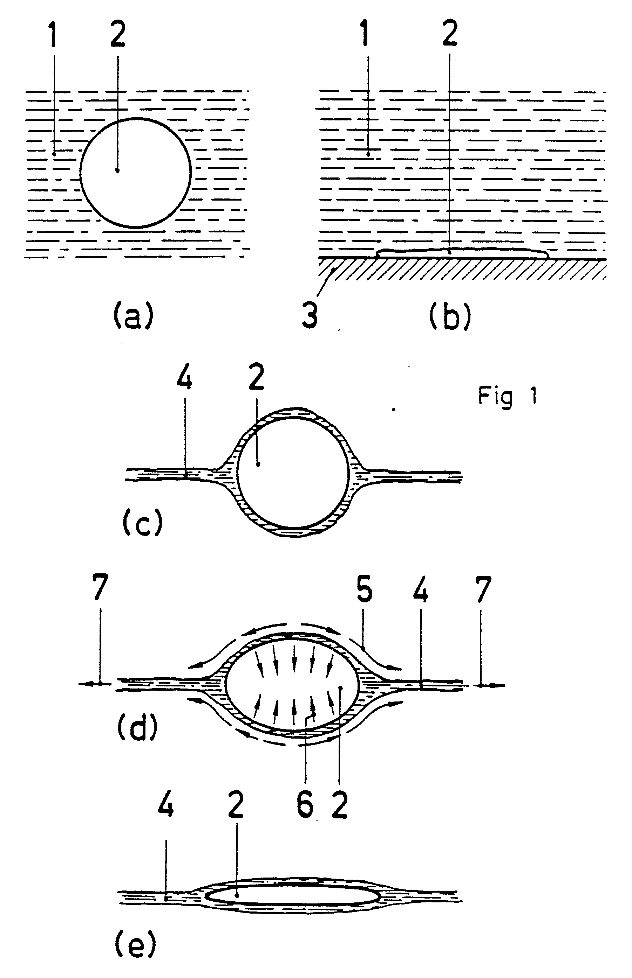

Figure 1a shows a cell (2) floating freely in bulk medium (1).

When in suspension the cell (2) is more or less spherical in shape.

Figure 1b shows a cell (2) which is anchored to a suitable solid surface (3). In this anchoraged state the cell (2) is spread out by attractive forces acting between the cell (2) and the solid surface (3).

Figure 1c shows a cell (2) trapped within a thin film of medium (4).

Placed in this thin film (4) from the bulk medium (1) the cell (2) is initially spherical in shape. Figure 1d and e show how surface tension forces (5) tend to minimize the protrusion caused by the cell leading to a flattened cell (2). The degree of flattening depends on the equilibrium "stiffness" of the cell (2) and the magnitude of the resultant "squeeze" stresses (6) induced by film tension (7). The dynamics of the spreading out of the cells (2) depends on the ratio between viscous and elastic components of cell resistance to shape distortion. Usually the elastic component of resistance (stiffness) is very small, and very weak stresses, given enough time, will spread out the cell and trigger its division cycle.

Various spatial organisations of the cell growth film are possible. The film may depend on a solid support or be self supporting.

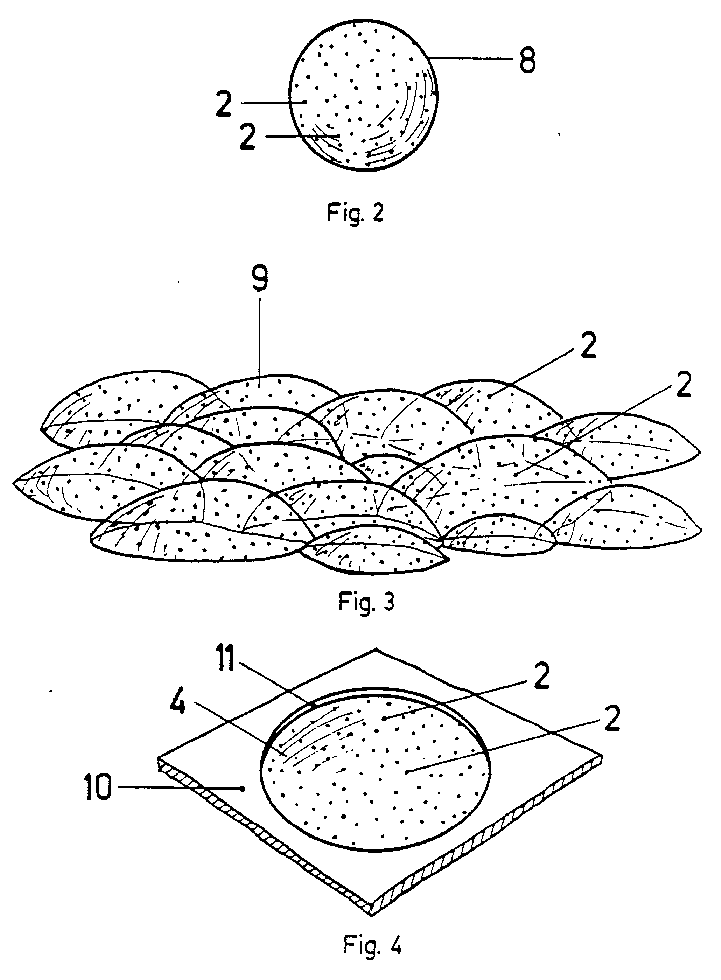

Figure 2 shows the simplest form that unsupported films may take—that of a sphere or "bubble" (8). A colony of cells is grown in a multitude of such bubbles, maintained, as described below, in a chamber of controlled environment.

Figure 3 shows an interconnected, cell supporting film in the form of a foam (9). As in the case of bubbles, the foam is maintained in a specially designed growth chamber.

Figure 4 shows a solid frame which supports thin growth films (4) over a multitude of perforations (11). A similar effect can be achieved by frames constructed of nets.

Addition of foetal calf serum (still required for most cell lines) makes the growth medium suitable for film formation. If necessary, film stability may be improved by suitable additives as disclosed in the British Patent Specification No. 1,525,022.

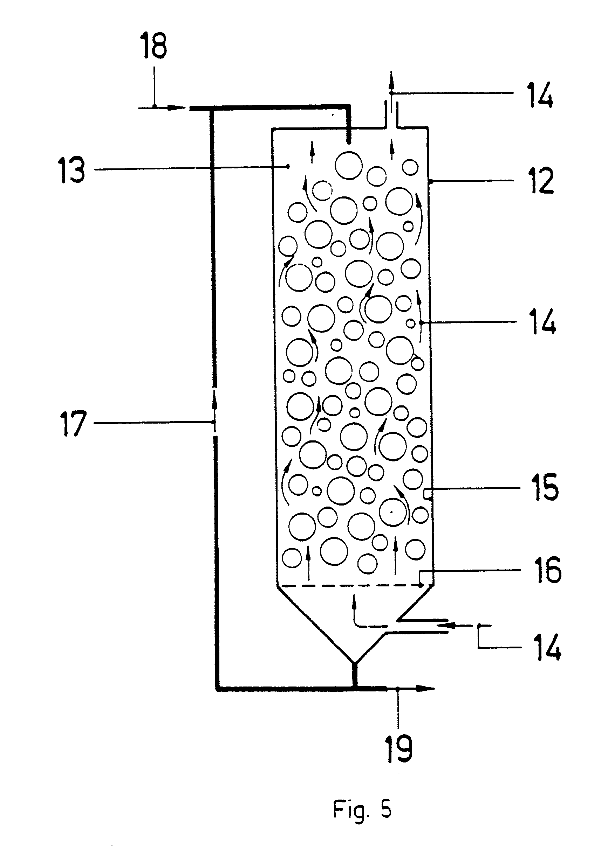

Figure 5 shows the basic features of a growth chamber (12) for maintenance of bubble cell colonies. Bubbles are formed from the cell-containing medium in the upper section (13) of the chamber (12) and are slowly descending against a stream (14) of 100% humid air of regulated temperature and composition. Each bubble carries an electrical charge (excess of a particular ion imposed at bubble formation) sufficient to keep them apart as they descend. The vertical walls (15) of the chamber (12) are also charged electrically. Furthermore the control of descent of the bubbles can also be effected or supplemented by an appropriate electrical field. Once the bubbles reach the bottom (16), they burst and the medium with the cells is circulated back (as represented by arrow (17)) to the top of the chamber (12) and into new bubbles. The medium is continuously replenished, as represented by arrow (18). Cell population is kept at optimum by cell removal, as represented by arrow (19), at a rate equal to the growth rate. Descent or "bubble time" must be longer than recirculation time, so that recirculation does not allow a cell to round up once it was spread out during "bubble time".

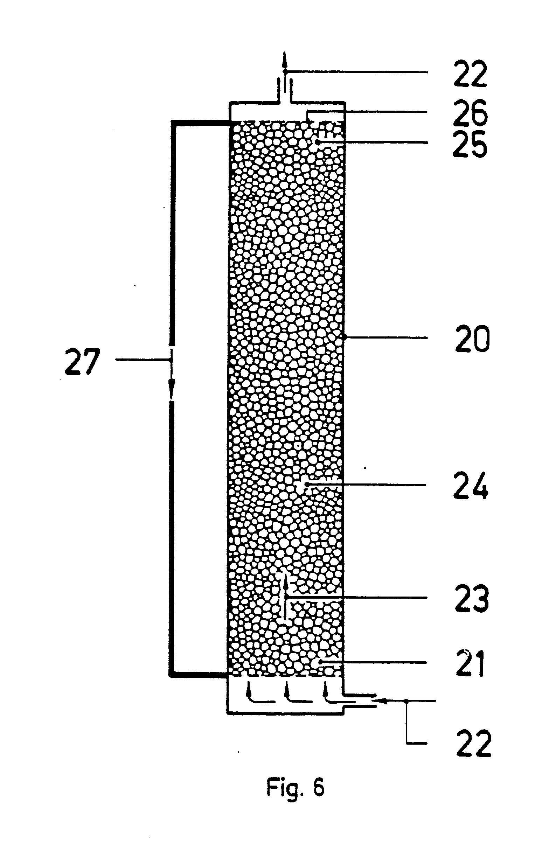

Figure 6 shows a chamber (20) for cell incubation in films of foam. The foam is continuously formed at the bottom (21) of the chamber (20) by a flow of air (22) of controlled composition, temperature and 100% humidity in order to avoid evaporation of the medium. The foam ascends as represented by arrow (23) within the chamber (20) at the rate determined by the air flow intensity. This should be very low to keep the foam structure (24) as stable as possible. At the top of the chamber (25) the foam breaks or is broken against a suitable barrier (26) and the medium with the cells is circulated back, as represented by arrow (27) to the bottom of the chamber (21) and reconverted into foam. Again the "foam time" should be much longer than the time needed for recirculation to avoid cell rounding. The efficiency of space utilisation in this particular configuration of growth film is much higher than in the case of bubble colonies.

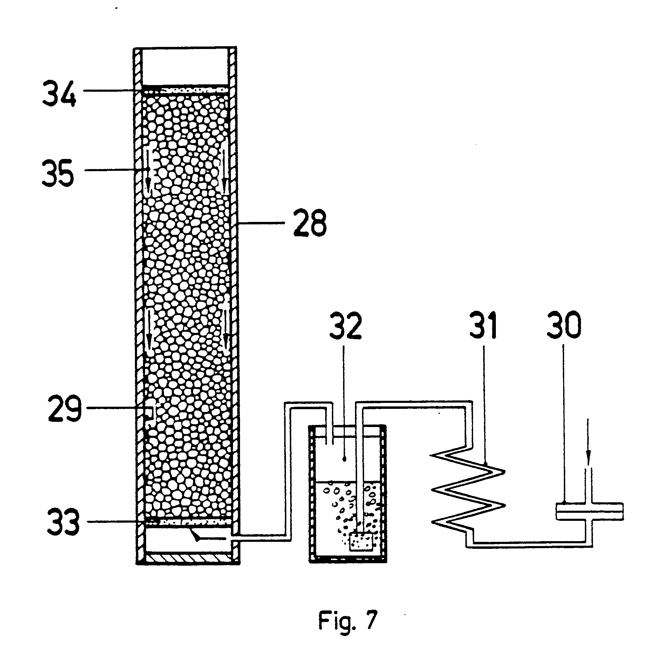

Figure 7 shows a laboratory scale apparatus consisting of a simple glass tube (28). All solid surfaces contacted by the medium are siliconized (29) in order to avoid cell anchorage. Air with an increased content of carbon dioxide for pH control is sterilized by filtration (30), heated up to the incubation temperature (31), saturated with moisture (32) and then introduced through a teflon filter (33) at the bottom of the chamber.

The hydrophobic teflon filter (33) prevents the growth medium with cells from running out. The foam is formed from any medium collected on top of the filter and slowly pushed upwards. A foam breaker (34) consisting of a teflon filter at the top of the tube allows air to escape while the medium runs down the walls, as represented by arrow (35) to the bottom of the tube where it is reconverted into foam.

## Claims

1. Method for the mass growth of anchorage-dependent cells characterized in that the cells (2) are grown in a thin film of nutrient medium (4).

2. Method according to claim 1, wherein the thin film is a bubble (8).

3. Method according to claim 1, wherein the thin film is a foam (9).

4. Method according to one of the claims 1 to 3, characterized in that the cells (2) are subjected to a recirculation process whereby the cells (2) in the thin film of nutrient medium (4) are recirculated to the bulk medium (1) for replenishing and then placed again in a thin film of nutrient medium (4).

5. Method according to claim 4, characterized in that the recirculation process is continuous.

6. Apparatus for carrying out the method according to one of the claims 1 to 5, comprising a growth chamber (12) for the maintenance of bubble cell colonies, said growth chamber (12) comprising a circuit (17) for recirculation of the bulk medium (1) producing continuously a multitude of bubbles descending from the top to the bottom (16) of the chamber (12) and an inlet at the bottom (16) of the chamber (12) and an outlet at the top of the chamber (12) for producing a counter stream of humid air and prolonging the descent time of the bubbles (8).

7. Apparatus for carrying out the method according to one of the claims 1 to 5 comprising a foam growth chamber (20) for cell incubation in films of foam, said chamber (20) comprising a foam producing section at the bottom (21) of the chamber (20) and a foam breaking section at the top (25) of the chamber (20) and a circuit (27) for recirculation of the bulk medium.

8. Apparatus according to claim 7, wherein the foam producing section comprises a filter, preferably from teflon, through which a flow of air (22) is introduced from the bottom of the chamber (20).

9. Apparatus according to one of the claims 6 to 8, wherein the solid surfaces are siliconized completely or partially.

**Patentansprüche**

1. Verfahren zur Massenkultur von trägerschichtabhängigen Zellen, dadurch gekennzeichnet, dass die Zellen (2) in einer dünnen Schicht von Nahrlösung (4) gezüchtet werden.

2. Verfahren nach Anspruch 1, dadurch gekennzeichnet, dass die dünne Schicht eine Blase (8) ist.

3. Verfahren nach Anspruch 1, dadurch gekennzeichnet, dass die dünne Schicht ein Schaum (9) ist.

4. Verfahren nach einem der Ansprüche 1 bis 3, dadurch gekennzeichnet, dass die Zellen (2) einem Kreislaufprozess unterworfen werden, wobei die Zellen (2) in der dünnen Schicht von Nahrlösung (4) zur Vorratslösung (1) zurückgeführt werden um regeneriert zu werden und dann wieder in die dünne Schicht der Nährlösung (4) gebracht werden.

5. Verfahren nach Anspruch 4, dadurch gekennzeichnet, dass der Kreislaufprozess kontinuierlich ist.

6. Vorrichtung zur Durchführung des Verfahrens nach einem der Ansprüche 1 bis 5, dadurch gekennzeichnet, dass die Vorrichtung eine Reaktionskammer (12) zur Aufrechterhaltung von blasenförmigen Zellkolonien umfasst, wobei diese Reaktionskammer (12) eine Leitung (17) zur Rückführung der Vorratslösung (1) unter kontinuierlicher Bildung einer Vielzahl von Blasen, die vom Dach zum Boden (16) der Reaktionskammer (12) herunterschweben, einen Einlass am Boden (16) der Reaktionskammer (12), sowie einen Auslass am Dach der Reaktionskammer (12) zur Erzeugung einer Gegenströmung von feuchter Luft zwecks Verlängerung der Schwebezeit der Blasen (8) aufweist.

7. Vorrichtung zur Durchführung des Verfahrens nach einem der Ansprüche 1 bis 5, gekennzeichnet durch eine Schaumerzeugungs-Kammer (20) zur Zellinkubation in dünnen Schaumschichten, wobei die Kammer (20) am ihrem Boden (21) eine schaumerzeugende Zone und an ihrem Dach (25) eine schaumbrechende Zone aufweist, sowie eine Leitung (27) zur Rückführung der Vorratslösung (1).

8. Vorrichtung nach Anspruch 7, dadurch gekennzeichnet, dass die schaumbildende Zone einen Filter, vorzugsweise aus Teflon, umfasst, durch den ein Luftstrom (22) vom Boden der Kammer (20) her eingeführt wird.

9. Vorrichtung nach einem der Ansprüche 6 bis 8, dadurch gekennzeichnet, dass die festen Oberflächen partiell oder vollständig silikonisiert sind.

**Revendications**

1. Procédé pour la culture en masse de cellules dépendant d'un ancrage, caractérisé en ce que les cellules (2) sont cultivées en une couche mince de liquid nutritif (4).

2. Procédé selon la revendication 1, caractérisé en ce que la couche mince est une bulle (8).

3. Procédé selon la revendication 1, caractérisé en ce que la couche mince est une mousse (9).

4. Procédé selon une des revendications 1 à 3, caractérisé en ce que les cellules (2) sont soumises à un procès de recyclage, les cellules (2) dans la couche mince de liquid nutritif (4) étant recyclées au liquid nutritif (1) pour être régénérées et ensuite placées de nouveau dans une couche mince de liquid nutritif (4).

5. Procédé selon la revendication 4, caractérisé en ce que le procés de recyclage est à action continue.

6. Appareil pour la mise en oeuvre du procédé selon une des revendications 1 à 5, caractérisé en ce qu'il comprend une chambre de réaction (12) pour le maintien de colonies de cellules en forme de bulles, cette chambre de réaction (12) comprenant une conduite (17) pour le recyclage du liquid nutritif (1) produisant de manière continue une multitude de bulles qui descendent du plafond au fond (16) de la chambre (12), une entrée au fond (16) de la chambre (12) et une sortie au plafond de la chambre (12) pour générer un contre-courant d'air humide qui prolonge la durée de descente des bulles (8).

7. Appareil pour la mise en oeuvre du procédé selon une des revendications 1 à 5, caractérisé en ce qu'il comprend une chambre de production de mousse (20) pour l'incubation de cellules en couches. minces de mousse, cette chambre (20) comprenant une zone de production de mousse au fond (21) de la chambre (20) et une zone anti-mousse au plafond (25) de la chambre (20) et une conduite (27) pour le recyclage du liquid nutritif (1).

8. Appareil selon la revendication 7, caractérisé en ce que la zone de production de mousse comprend un filtre, préférablement en téflon, à travers duquel un courant d'air (22) est introduit du fond de la chambre (20).

9. Appareil selon une des revendications 6 à 8, caractérisé en ce que les surfaces solides son partiellement ou complètement traitées au silicone.

1   2

(a)

1   2

3   (b)

4   2

Fig 1

(c)

7   5 4 7

(d)

6 2

4 2

(e)

Fig. 2

Fig. 3

Fig. 4

Fig. 5

Fig. 6

Fig. 7